# EUROPEAN PATENT SPECIFICATION

(11) **EP 1 592 402 B1**
(45) Date of publication and mention of the grant of the patent: **20.03.2013**
(21) Application number: 04704035.7
(22) Date of filing: 21.01.2004
(51) Int. Cl.: A61K 9/48, A61K 47/06, A61K 9/00

(54) **DISPERSION OF TASTE MASKED CRYSTALS OR GRANULES OF ACTIVE SUBSTANCES, CHEWABLE SOFT CAPSULES FILLED WITH SAID DISPERSION, AND PROCESS FOR PREPARING SAME**
DISPERSION VON GESCHMACKSMASKIERTEN KRISTALLEN ODER GRANULATEN VON WIRKSTOFFEN, MIT DER DISPERSION GEFÜLLTE WEICHE KAUKAPSELN UND VERFAHREN ZU IHRER HERSTELLUNG
DISPERSION DE CRISTAUX OU DE GRANULES DE SUBSTANCE ACTIVE AU GOUT MASQUE, CAPSULES MOLLES MASTICABLES EN ETANT REMPLIES, ET LEUR PROCEDE DE PREPARATION

(30) Priority: 24.01.2003 FR 0300824
(43) Date of publication of application: 09.11.2005
(73) Proprietor: R.P. Scherer Technologies, LLC, Carson City, NV 89706 (US)
(72) Inventor: MEISSONNIER, Julien, F-67610 La Wantzenau (FR); ROSE, Fabrice, F-67540 Ostwald (FR); BOHN, Marie Madeleine, F-67480 Auenheim (FR)
(74) Representative: Dunleavy, Kevin James
(86) International application number: PCT/US2004/001463
(87) International publication number: WO 2004/066925

(56) References cited:
- EP-A- 1 163 901
- WO-A-94/08576
- WO-A-95/00123
- WO-A-96/36320
- WO-A-02/096392
- US-A- 4 851 226
- US-A- 5 019 563
- US-A- 5 320 855
- DATABASE WPI Week 199327 Thomson Scientific, London, GB; AN 1993-216649 XP002506386 & JP 05 139959 A (EISAI CO LTD) 8 June 1993 (1993-06-08)
- DATABASE WPI Section Ch, Week 199342 Thomson Scientific, London, GB; Class B07, AN 1993-331368 XP002255247 & JP 05 238954 A (TOYO KAPUSERU CAPSULE KK) 17 September 1993 (1993-09-17)
- ANAND ET AL: "The latest trends in the taste assessment of pharmaceuticals", DRUG DISCOVERY TODAY, ELSEVIER, RAHWAY, NJ, US, vol. 12, no. 5-6, 27 February 2007 (2007-02-27), pages 257-265, XP005908117, ISSN: 1359-6446, DOI: 10.1016/J.DRUDIS.2007.01.010
- KRZYSZTOF SOLLOHUB ET AL: 'Taste masking of roxithromycin by spray drying technique.' ACTA POLONIAE PHARMACEUTICA vol. 68, no. 4, 01 January 2011, pages 601 - 604, XP055020749 ISSN: 0001-6837

## Description

### TECHNICAL FIELD

This invention relates to a new dosage form that provides a fast dissolving soft capsule that contains a liquid fill that comprises a dispersion of taste masked crystals or granules of a bad tasting active ingredient. In particular, said dosage form is a dispersion of crystals or granules of active substances in a lipophilic vehicle, said crystals or granules being coated by a coating for taste masking purposes. The invention relates also to chewable soft capsules that are fast dissolving in the mouth and contain said dispersion and to a process for preparing the novel dosage form.

### BACKGROUND OF THE INVENTION

The oral administration of medicines, and in particular medicines that have an unpleasant taste, is very difficult in some categories of patients, in particular infants, children, and patients who have difficulty in swallowing. Such difficulties lead to poor treatment compliance. Various dosage forms have been developed to overcome the problem of bad taste in oral formulations.

Examples of approaches considered to overcome this problem include coating of crystals or granules of active substance, complexing (inclusion complexes) of the active substance in cyclodextrine type molecules as described in U.S. Patent Nos. 5,019,563 and 5,024,997, addition of agents that cause a slight localized effervescence as described in U.S. Patent Nos. 5,180,590 and 5,262,179, addition of a very large quantity of flavorings and maintenance of a certain pH environment as described in U.S. Patent No. 4,975,465, modification of the crystalline form of the active substance as described in U.S. Patent No. 5,466,865, s use of a mixture of ibuprofen and its aluminum salt as described in U.S. Patent No. 4,831,058, and encapsulation of the active substance as described in U.S. Patent Nos. 5,814,332 and 5,653,993.

EP-A 1 163 901 is directed to improving the properties of chewable soft capsules including reducing the bitter taste of the fill material, alleviating the unpleasant sensation typical of capsule shells by causing the fill and shell materials to homogenize with each other in the mouth when chewing the capsule, and reducing the sensation of oiliness of the oil base. This document also discloses two methods for reducing the bitterness of a drug in the fill material: (a) restricting the amount of drug in the capsule to a low level or adding a bitter tasting material to the fill material such as bitter chocolate and/or cacao powder.

International publication number WO 94/08576 A1 is directed to alleviating the bitter taste of ranitidine in capsules. This publication discloses coating the ranitidine, or a core containing the ranitidine, with a lipid to form lipid-coated ranitidine or a lipid-coated ranitidine-containing core and dispersing the lipid-coated ranitidine or ranitidine-containing core in a non-aqueous vehicle.

More specifically, for oral formulations intended for children, numerous suspension dosage forms exist. These require the development of special coated particles whose coating for taste masking purposes must, on the one hand, resist the aqueous media during storage and, on the other, be able to disintegrate very quickly once swallowed to obtain a satisfactory bioavailability in viva of the active substance.

Development of useful coatings is difficult, as it requires the use of large amounts of expensive polymers, coating agents and mainly pH- depending polymers.

As coating levels are high, drug content is limited as particle size is a factor, which must be addressed for an acceptable mouthfeel. Furthermore, the prior art preparations have problems of stability, and loss of taste masking can occur after long periods of storage.

Fast-dissolving tablets are also available. The fast dissolving tablets can be manufactured through a process requiring the lyophilization of an aqueous solution, as described in GB Patent No. 1548022. The production of such tablets involves delicate preparation of the taste masking coating, which must be resistant to the aqueous medium used in the production of the dosage form and breakdown rapidly once swallowed so that the active substance becomes available.

Tablets that disintegrate quickly in the mouth with or without a chewing action have also been proposed. See for example International Application Nos. WO 99/04763 and WO 00/51568. However, the production of such solid tablets also involves delicate preparation of the coating for taste masking purposes. Here the coating must be resistant to the pressure applied by chewing and breakdown rapidly once swallowed. In some cases, these coating materials for solid tablets must be elastic and thick in order to survive a compression operation (to form the tablet) and must also dissolve rapidly after swallowing. Furthermore, these tablets have a tendency to leave a doughy, granular or so-called sandy feeling in the mouth, which can be unpleasant. This is due to the particle size distribution of the coated crystals and/or granulates and to the absence of a liquid vehicle, except for saliva.

Furthermore, soft capsules containing an active substance with an unpleasant taste, in particular ibuprofen, have been described in International Application Nos. WO 88/02625, WO 93/11753, WO 94/14423 and WO 02/17855. In these capsules, the active substance is placed in solution in a hydrophilic vehicle. With the solubilization of the active substance having already been achieved, *in vivo* release is predicted to be faster. However, these capsules are intended to be swallowed and often require the use of a glass of water for people having difficulties in swallowing. These dosage forms cannot be chewed since the fill formulation combines components, which can present a very unpleasant taste, and in some cases a burning sensation. Fill components, such as polyethylene glycol and ibuprofen, present strong bitterness and a burning effect in the mouth.

There is a real need for pharmaceutical dosage forms for oral administration that are easy for all types of patients to take which are suitable for active substances with an unpleasant taste. The dosage form according to the present invention does not leave a doughy or granular feeling in the mouth. The present invention also provides dosage forms that are stable throughout a storage period, that allows a large quantity of active substance to be taken in a single administration and which provides for satisfactory release *in vivo* of the active substance.

### SUMMARY OF THE INVENTION

The present inventors have surprisingly and unexpectedly found that an improved oral dosage form that comprises in a preferred embodiment a chewable soft capsule that rapidly dissolves in the mouth that is filled with a dispersion of crystals or granules of active substance in a lipophilic vehicle which has a solubulizing power for the active substance of less than 1.5 times the active substance concentration for which a taste is detected in water, the said crystals or granules being coated by a coating for taste making purposes which is a hydrophilic coating and/or lipophilic coating selected from the coating materials listed in claim 1. Said dispersion can be filled in any type of containers, for example, conventional soft capsules, chewable soft or fast dissolving soft gelatin capsules, ampoules, sachets, stick-packs and the like. The lipophilic vehicle has a solubulizing power for the active substance of less than 1.5 times the active substance concentration for which a taste is detected in water. According to another embodiment of the present invention, said dispersion can be encapsulated into fast dispersible chewable soft capsules comprising a chewable outer envelope.

In the present invention, the term "coating for taste masking purposes" means any coating that prevents a quantity of the active substance that is detectable by the taste buds from coming into contact with said taste buds in the buccal cavity when the capsule is ruptured, and which is a hydrophilic coating and/or lipophilic coating, and is selected from ethylcellulose, hydroxyethylcellulose, hydroxypropylmethylcellulose, hydroxypropylmethyl cellulose phthalate, methacrylic copolymer, polyethylene glycol behenate, glycerol palmitostearate, glycerol stearate, cetyl palmitate, glyceric macrogols, beeswaxes and gelucires, and mixtures thereof. In the dispersion according to the invention, the granules or crystals coated with at least one taste-masking agent are in suspension in a lipophilic vehicle which has a solubulizing power for the active substance of less than 1.5 times the active substance concentration for which a taste is detected in water.

The present invention offers advantages over previous systems as lower stress is applied to the coated particles of active ingredient. Further, the coating does not need to resist an aqueous fill, as it will not be suspended in an aqueous based medium. Further, in the dosage form according to the present invention, the coating does not need to withstand a compression operation that is used to prepare tablets. Further, the lipophilic fill provides an "over-coating" of the taste masked active. Thus, when the lipophilic fill is released from the capsule (or any other unit dose envisaged) in the mouth, a thin lipophilic layer surrounds the coated particles of active and provides additional isolation of the taste buds.

Taste perception is mainly linked to the amount of drug substance in solution or free in the lipophilic vehicle. One benefit of the present invention resides in the use of hydrophilic and/or lipophilic coatings, which are easy to apply and are used at lower coating levels, i.e. a lower weight ratio of coating/active substance, than conventionally used.

Furthermore, the present invention is particularly suited for highly water soluble drug substances, which present a lower solubility in most lipidic vehicles. These drugs are also difficult to taste-mask using standard dosage forms due to the fact they will quickly dissolve in saliva and reach the taste buds. In contrast, these drugs can be used without difficulty and provide very satisfactory results in the dispersions according to the invention.

One aspect of the invention resides in the selection of the lipophilic vehicle for the dispersion of the coated active ingredient. The lipophilic vehicle is chosen in order to have a limited solubilization power for the active and/or for at least one of the components of the taste mask coating.

It should be noted that an acceptable degree of solubilization of the drug or active substance is a function of drug loading and taste of the active. Taste can be quantified by determination of the taste perception limit of the drug in a liquid vehicle. In the case of an active with a very unpleasant taste, like ibuprofen for example, the lipophilic vehicle will be selected so as not to solubilize or minimize the solubilization of the ibuprofen and the coating components. On the other hand, in the case of an active with a less unpleasant taste, a certain degree of solubilization of the active and/or of the coating may be acceptable.

In other words, the concentration of free active substance in the lipophilic vehicle should be, at most, about 1.5 times the active substance concentration for which a taste is detected in water, and preferably, at most, about 1.0 times and even more preferably, at most, about 0.5 times. For example, if the drug of interest can be detected at 1.0 mg/ml in water, no more than 1.5 mg/ml should be the solubility of the free active substance in the lipophilic vehicle.

The dispersion according to the invention is very stable during storage, i.e. the organoleptic properties of the dispersion are maintained during at least 3 months, preferably at least 1 year and even more preferably during at least 2 years.

### DETAILED DESCRIPTION OF THE PREFERRED EMBODIMENTS

The lipophilic vehicle is selected from the group comprising digestible oils and mineral oils and mixtures thereof. In the present invention, "digestible oil" means an oil that is likely to undergo de-esterification in the presence of pancreatic lipase *in vivo* under normal physiological conditions.

The digestible oils are selected from the group containing in particular vegetable and animal oils, di-, tri- or monoglycerides of fatty acids, lipolyzing oils, full or partial esters of medium chain (C₈-C₁₂) or long chain (C₁₄-C₂₂)fatty acids, with mono-, di- or polyalcohols of low molecular weight (having up to 6 carbon atoms), and mixtures thereof.

Specific examples of vegetable oils that can be used as a lipophilic vehicle include soya, maize, olive, cotton seed, peanut, sunflower, coconut, palm, rape, grape seed, wheat germ, sesame, avocado, almond and apricot stone oils and mixtures thereof.

Examples of animal oils that can be used as a lipophilic vehicle of the dispersion according to the invention include in particular fish liver oils, shark oil and mink oil.

Specific examples of triglycerides that can be used as a lipophilic vehicle in the dispersion according to the invention are in particular those containing saturated C₆-C₁₂ fatty acids, in particular capric and/or caprylic acid triglycerides, such as Miglyol^{®}810 and Miglyol^{®}812 sold by Sasol, Neobee^{®}M5 and Neobee^{®}0 sold by Stepan Europe, and Captex^{®}300, Captex^{®}355 and Captex^{®}8000, sold by Abitec.

The mineral oils are selected from the group comprising paraffin, light waxes, Vaseline, silicon oil, such as dimethicone, mixtures of silicon oil and colloidal silica such as simethicone, and mixtures thereof.

The lipophilic vehicle can also contain further additives selected from the group comprising thickeners and/or dispersants, sweeteners, flavorings, colorants, effervescent agents, super-disintegrants, lipophilic surfactants, hydrophilic surfactants, hydrosoluble agents promoting *in vivo* dispersion and mixtures thereof.

The present invention is especially suited to active substances that have an unpleasant taste and combinations of such actives. Such active substances can be food substances such as vitamins, mineral salts, oligo-elements, herbal extracts or also pharmaceutical substances selected from the group comprising analgesics such as aspirin, acetaminophen, acetaminophen with caffeine; non-steroidal antiinflammatories such as ibuprofen, diclofenac, aceclofenac, fenoprofen, flurbiprofen, ketoprofen, naproxen and its alkaline metal salts, nimesulide, piroxicam and its salts; H₂ antagonists such as cimetidine, ranitidine hydrochloride, famotidine, nizatidine, ebrotidine, mifentidine, roxatidine, pisatidine and aceroxatidine; antiallergenic agents such as codeine and its hydrochloride, codeine and its phosphate, ebastine, clemastine and its fumarate, azatidine and its maleate, hydroxyzine and its pamoate and its hydrochlorides, chlorpheniramine and its maleates and tannates, pseudoephidrine and its sulphates and hydrochlorides, bromopheniramine and its maleate, loratidine, phenylephrine and its tannates and hydrochlorides, methscopolamine and its nitrates, phenylpropanolamine and its hydrochlorides, bromopheniramine and its maleate, terfenadine, acrivastine, astemizole, cetirizine and its hydrochloride, phenindamine and its tartrate, tripelennamine and its hydrochloride, cyproheptadine and its hydrochloride, cyproheptadine and its hydrochloride, promethazine and its hydrochloride, pyrilamine and its hydrochlorides and tannates; anti-migraine agents such as divalproex and its alkaline metal salts, timolol and its maleate, propanol and its halogen hydrates, ergotamine and its tartrate, caffeine, elitriptan, sumatriptan and its succinate, and active substances of the same therapeutic class; dihydroergotamine, its hydrogenates and mesylates, methsergide and its maleate, isomethepten mucate, dichloralphenazone; antiemitics such as meclizine and its hydrochloride, hydroxyzine and its hydrochloride and pamoate, diphenhydramine and its hydrochlorides; prochlorperazine and its maleate, benzquinamide and its hydrochloride, granisetron and its hydrochloride, dronabinol, bismuth subsalicylate, promethazine and its hydrochloride, metoclopramide and its halides/hydrates, chlorpromazine, trimethobenzamide and its hydrochloride, thiethylperazine and its maleate, scopolamine, perphenazine, ondansetron and its hydrochloride ; antidiarrheals such as loperamide; antihistamines such as seldane, hismanal, relafen, tavist; antitussives, decongestants, axiolytics such as xanax; antipsychotics such as clozaril and ilaldon; antiemetics such as kytril and cesamet; bronchodilators such as bentolin and proventil ; antidepressants such as prozac, zoloft, and paxil; ACE-inhibitors such as vasotec, capoten and zestril; anti-alzheimers agents such as nicergoline; and Ca₁₁ -antagonists such as procardia, adalat, and calan; anticholesterolemics such as lovastatin and pravastatin; cold and cough products such as dextromethorphan and its bromhydrate salts and guaifenesin and its hydrochloride salts; CoX-2 inhibitors; antiepileptic compounds; 5HT inhibitors such as sildenafil; proton pump inhibitors such omeprazole, pantoprazole, lanzoprazole, terbinafine; and macrolides such as chlarithromycine, roxythromicin, azythromycin and ketolides such as telithromycin, beta-lactamin, cephalosporin, fluoroquinolone, and quetiapine.

This list includes corresponding salts such as amino acids, metal salts and the like. These active salts should be selected regarding their compatibility with the coating/taste masking polymers, the taste in the lipophilic fill and their bioavailability linked to rapid dissolution in the stomach.

The concentration of active substance in the dispersion depends on the container in which said dispersion is intended to be encapsulated. It will be apparent to a person skilled in the art that said concentration will be lower in stick-packs or in sachets than in capsules.

The concentration of the active in a soft capsule is at most 75%, preferably between 5 and 50%, and even more preferably between 15 and 40% by weight.

The active substance may be granulated prior to being coated, particularly in the case where the crystals of the active material are too small to be directly coated or to give the active substance improved solubilization and disintegration properties once the taste masking coating is removed. Granulation is performed by any suitable process well known to a person skilled in the art.

According to a particular embodiment, the active substance may be granulated on a neutral core with the help of a solution or dispersion containing solubilization agents, granulation agents, binders and possibly sweeteners, flavorings and colorants.

If it is desired to increase the dissolution *in vivo* of the active substance, it is also possible to introduce into the granules additional excipients selected from the group comprising effervescent agents, super disintegrants, lipophilic surfactants, hydrophilic surfactants, hydrosoluble agents promoting dispersion, and mixtures thereof.

As the invention is particularly suitable for the formulation of active substances that have a bad taste, the crystals or granules of active substances are provided with a coating for taste masking purposes. This coating being preferentially hydrophilic based in order to achieve the faster in-vivo release.

Useful coatings are described in the following references: International Application No. WO 01/03672, and U.S. Patent Nos. 5,320,855 and 5,552,152.

In preferred embodiments, the coatings consist of a mixture of: ethyl cellulose and hydroxypropylmethylcellulose as described in International Application No. WO 01/03672, or a mixture of hydroxyethylcellulose and hydroxypropylmethylcellulose as described in U.S. Patent No. 5,320,855. Other useful coatings include a methacrylic copolymer with sufficient elasticity as described in U.S. Patent No. 5,552,152, waxes, glyceric macrogols, in particular when these are applied by the so-called "hot-melt coating" process which is a hot spraying of a lipophilic or lipidic based material using fluidized air bed equipment onto a fluidized substrate. See U.S. Patent No. 6,194,005 for further details. Representative materials include glycerol and/or polyethylene glycol behenate (Compricoat^{®}, Compritol^{®}888 ATO, HD5 ATO), glycerol palmitostearate (Precirol^{®}ATO 5), glycerol stearate (Precirol^{®}WL2155 ATO) cetyl palmitate (Precifac^{®}ATO), Macrogol^{®}, beeswaxes, gelucires such as glycerol and PEG-32 laurate, glycerol and PEG-32 palmitostearate, glycerol and PEG-32 stearate.

The coating may also contain further additives selected from the group comprising colorants, sweeteners, flavorings, lipophilic surfactants, hydrophilic surfactants, hydrosoluble agents promoting *in vivo* dispersion, and mixtures thereof.

The coating can be performed by any method known to a person skilled in the art, in particular by coacervation or spraying such as fluidized air bed coating as described in particular in International Application No. WO 01/03672 and U.S. Patent No. 5,552,152, rotogranulation, as described in particular in U.S. Patent No. 5,320,855, and by hot spraying of lipidic or lipophilic compounds, that is to say by the so-called hot-melt coating process as described in U.S. Patent No. 6,194,005.

The coating for taste masking purposes can also be adapted to modify the *in vivo* dissolution profile of the active ingredient.

This taste masking coating represents between 2 and 70%, preferably between 5 and 50%, and more preferably between 5 and 30% by weight of the total weight of the coated crystals or granules.

According to a preferred embodiment of the invention, the coated crystals or granules of active substance have an average particle size of less than 300 µm, preferably between 5 and 180 µm, and more preferably between 50 and 150 µm.

Average particle size of the coated crystal and/or granules should be below 300 µm in order to provide good palatability and mouthfeel. Moreover, particle size distribution has an effect on container seal formation, in particular capsule seal formation. Encapsulation of larger particles can create untight seals during routine manufacturing operations.

As it will be obvious for the person skilled in the art, any additive added in the lipophilic vehicle must be added in proportions that will not affect coating integrity and not increase drug substance solubility in the final dispersion.

The effervescent agents that can be included in the granules or also the lipophilic vehicle, allow an increase in the dissolution of the active substance *in vivo*, in particular by localized modification of the pH, and by swelling of the particles. However, when the dispersion is intended to be filled into soft capsules, the use of effervescent agents in the lipophilic vehicle must be avoided.

Such effervescent agents are agents that are capable of releasing a gas, and these are, in particular, acid-base pairings. The acid is selected from the group comprising, in particular, tartaric acid, citric acid, maleic acid, fumaric acid, malic acid, adipic acid, succinic acid, lactic acid, glycolic acid, alpha hydroxy acids, ascorbic acid and amino acids, as well as their salts and derivatives, and mixtures thereof. The alkaline agent is selected from the group comprising carbonate of potassium, lithium, sodium, calcium, or ammonium, or carbonate of L-lysine, carbonate of arginine, carbonate of sodic glycone, sodic carbonates of amino acids, anhydrous sodium perborate, effervescent perborate, sodic perborate monohydrate, percarbonate of sodium, sodium dichloroisocyanide, calcium hypochlorite, and mixtures thereof.

The respective quantities of acid agent and alkaline agent are adjusted so that the reaction between the alkaline agent and the protons released by the acid allow the generation of a sufficient quantity of gas to obtain a satisfactory effervescence.

The super-disintegrants included in the granules, and/or the coatings are selected from the group comprising cross-linked sodium carboxymethylcellulose referred to in the trade as croscarmellose, crospovidone, and mixtures thereof.

Thickeners and/or dispersant agents may be placed in the lipophilic vehicle to reduce any sedimentation of the coated particles. Representative agents include gums such as guar gum, xanthans, short chain oligomers, partially hydrogenated vegetable oils, hard fats, waxes such as beeswax, carnauba, and ampiphilic compounds such as soya lecithin in particular, colloidal silica, and mixtures thereof.

Lipophilic surfactants and the hydrophilic surfactants may be present in the granules, the coating and/or the lipophilic vehicle to facilitate the rupture of the taste masking coating. Representative surfactants are described in International Application No. WO 95/00561. Other surfactants include sucroesters that are esters of saccharose fatty acids, chosen from the group comprising saccharose distearates, saccharose mono-distearates, saccharose monopalmitate and mixtures thereof.

The dispersion of the active substance *in vivo* can also be improved by the addition of hydrosoluble agents to the granules, the coating and/or the vehicle. These agents are salts or buffers selected from the group comprising sodium chloride, potassium hydroxide, potassium citrate, potassium sorbate, sodium mono- or diphosphate, and mixtures thereof.

The colorants, flavorings and sweeteners that can be included in the lipophilic vehicles, granules or coatings according to the invention are those of conventional use in the food or pharmaceutical industry. These additives are added in sufficient quantities to give the desired taste and appearance properties and can easily be adapted by a professional.

Examples of colorants include azorubin, FCF brilliant blue, patent blue V, erythrosine, brown, yellow, black, red 4, yellow orange S iron oxides, quinoline yellow, FDC red, DC red 33, cochineal red A, timiron, titanium dioxide, FD8C red 40, FD8C green 3, curcumine, lactoflavin (riboflavin), tartrazine, amaranthine, indigotine (indigo carmine), chlorophyls, copper complexes of chlorophyls and chlorophyllins, brilliant acid green BS, caramel, brilliant black BN, carbo medicinalis vegetalis, carotenoids (alpha-, beta-, gamma-carotene, bixin, norbixin, capsanthin, capsorubine, lycopene, beta-apo-8'carotenal, ethyl ester of beta-apo-8' carotenoic acid), xanthophylls, (flavoxanthin, lutein, cryptoxanthin, rubixanthin, violoxanthin, rhodoxanthin, canthaxanthin), beetroot red, betanin, anthocyans, calcium, aluminum, or silver carbonate or mixtures thereof.

Examples of flavorings include essential oils, possibly in a dry form with a maltdodextrine or other base such as propylene glycol, pulegone, maltol, such as the essential oils of lemon, orange, menthol, fruit flavorings, aniseed, caramel, honey, liquorice, cream, various spices and combinations thereof with other flavorings, and mixtures thereof.

Examples of sweeteners include aspartame, saccharine, sodium saccharinate, acesulpham K, sucralose, neohesperidin hydrochloride, mannitol, xylitol, maltitol, sorbitol, sodium cyclamate, menthol and mixtures thereof.

According to a preferred embodiment of the invention, the dispersion comprises, as lipophilic vehicle, dimethicone in which there is dispersed coated granules of active substance, preferably ibuprofen, said coating comprising a mixture of ethylcellulose and hydroxypropylmethylcellulose, said coating representing 5% to 30% of the total weight of the granules, with the active substance representing between 5 and 50% by weight of the total weight of the dispersion, with at least one additive being dispersed in the dimethicone, said additive should not substantially affect the taste-masking effect of the coating and preferentially should improve the taste masking efficiency, and is selected from the group comprising colorants, flavorings and sweeteners.

The dispersion according to the invention can be presented as a syrup or, preferably, is intended to be filled in any type of containers, in particular in conventional, chewable or soft-gel type capsules, ampoules, sachets and stick-packs.

The present invention also concerns "chewable capsules" and fast dissolving capsules filled with the dispersion described above. The term "fast dissolving capsule" means a capsule, which disintegrates in the mouth without chewing in less than 30 seconds, i.e. the dispersion fill is released in the mouth in less than 30 seconds. The gelatin shell constituting the envelope of said capsule may disperse in the mouth in less than 120 seconds.

The term "chewable capsule" means a soft capsule that ruptures under the pressure exerted by the jaws or the tongue when it is placed in the buccal cavity. The residue obtained when the capsule ruptures disperse quickly in the mouth. Chewable soft capsules that are completely suitable for the formulations according to the invention are those described, for example, in International Application No. WO 95/00123.

One important aspect of the present invention resides in the fact that the inventive dispersions and soft capsules filled with those dispersions have excellent physical stability for at least 3 months, preferably for at least 1 year, and even more preferably for at least 2 years.

According to a particular embodiment of the chewable soft capsule according to the invention, the outer envelope of the chewable soft capsule comprises gelatin and a plasticizer, and at least a starch or amylum acetate compatible with the gelatin, with the gelatin forming a primary matrix for the plasticizer and the starch or amylum acetate forming a secondary matrix for the plasticizer.

According to a preferred embodiment, the outer envelope or capsule wall comprises between 18 and 30% gelatin, between 30 and 45% by weight of plasticizer, between 3 and 12% of starch or amylum acetate and up to 12%, preferably between 6 and 10%, of an unbleached starch and water to 100%, with these percentages being percentages by weight in relation to the total weight of the wall composition.

The unbleached starch acetate is preferably of potato origin. The gelatin could be of bovine, porcine, fish, poultry origin. The plasticizer is chosen from the group comprising polyols, in particular glycerol, xylitol, sorbitol, polyglycerol, non-crystallisable solutions of sorbitol, glucose, fructose and glucose syrups, and mixtures thereof. An advantageous plasticizer is a mixture of sorbitol, sorbitans, maltitol and mannitol sold under the brand name ANIDRISORB^{®} by Roquette.

The preferred plasticizer is glycerol and represents at least 30% by weight, preferably between 30 and 70% by weight, of the composition of the outer envelope (dry film).

The "crunchable" or "chewable" nature of the capsule can be modified by adding to the composition of the envelope an oil such as distilled coconut oil. The quantity of oil present in the envelope may be up to 15%, preferably not more than 10% and more preferably between 3 and 7%, typically 5%, by weight of the weight of the composition of the envelope preparation.

The outer envelope of the soft capsule according to the invention can also comprise sweeteners and flavorings, thereby giving the chewable soft capsule a more pleasant taste. It may also contain colorants in order to give said capsule a pleasant and attractive appearance.

The sweeteners, flavorings and colorants are those, which are described above in relation with the dispersion composition.

According to a particularly advantageous embodiment of the invention, the chewable soft capsule comprises:
an envelope with said envelope encapsulating a dispersion in dimethicone of active substance granules coated with a mixture of ethyl cellulose and hydroxypropylmethylcellulose, with the active substance representing between 5 and 50% by weight of the total weight of the dispersion, with at least one additive being dispersed in the dimethicone, said additive should not substantially affect the taste-masking effect of said mixture and is selected from the group consisting of colorants, flavorings and sweeteners.

The present invention is also concerned with the process for preparation of chewable soft capsules. This process comprises the steps of:
a) preparing a film for a capsule,
b) as necessary, preparation of granules of active substance;
c) coating crystals of active substance or granules prepared above using a coating for taste masking purposes;
d) dispersing additives in a lipophilic vehicle, wherein said lipophilic vehicles has a solubilizing pover for the active substance of less than 1.5 times the active substance concentration for which a taste is detected in water, and optionally milling of this;
e) dispersing coated crystals or granules in the lipophilic vehicle to form a dispersion;
f) filling of the capsules with said dispersion by the means of any known technique;
g) sealing of the capsules by the means of any known technique; and
h) drying of the capsules.

The step for preparation of the film for the capsule or outer envelope is performed according to the process described in International Application No. WO 95/00123. The granulation can be carried out by any of the conventional granulation processes, in particular dry granulation and wet granulation. Coating can be carried out by conventional means and preferably by fluidized air bed coating.

According to a preferred embodiment, the process comprises steps of:
a) preparing a film for a capsule;
b) coating of crystals of active substance, preferably ibuprofen, by spraying of a mixture of ethyl cellulose and hydroxypropylmethylcellulose dissolved in an organic solvent system;
c) dispersing colorants, sweeteners and flavorings in a lipophilic vehicle wherein said lipophilic vehicle has a solubulizing power for the active substance of less than 1.5 times the active substance concentration for which a taste is detected in detected in water and optionally milling of this;
d) dispersing the coated crystals or granules in the lipophilic vehicle with additives added;
e) filling the capsules with said dispersion by the means of any known technique;
f) sealing the capsules by the means of any known technique;

The invention is now described in a detailed manner using the following examples, which are intended solely to illustrate the invention and are not restrictive.

### EXAMPLES

### Example 1: Lipophilic Vehicles

In this example, lipophilic vehicles with different solubilizing powers for ibuprofen were investigated.

The solubility of ibuprofen at 25°C was measured using an HPLC method in the following lipophilic vehicles:
1) Medium chain triglycerides sold by SASOL under the brand name Mygliol^{®} 812N. The solubility was 65mg/ml.
2) Soya oil. The solubility was 44mg/ml.
3) Dimethicone 100 silicone oil from Rhodia. The solubility was 1 mg/ml.

Using a taste panel of two (2) persons, it was determined that, in aqueous buffer and lipophilic dispersions, the bitter taste of ibuprofen was detected at concentrations above 10 mg/ml. This taste limit was also confirmed in other lipophilic vehicles as well. Using the teachings of the present invention, dimethicone would be an acceptable vehicle. This was cross-checked by the following study. Three formulations were prepared using ibuprofen coated particles prepared by coating on a fluidized air bed.
Formulation 1: 80% by weight ibuprofen; and 20% by weight glycerol palmitostearate sold under the brand name PRECIROL^{®} ATO 5. The process used to obtain such particles is set forth in U.S. Patent No. 6,194,005. These coated particles were dispersed in the following four (4) lipophilic vehicles at a concentration of 25 wt. %:
   1) Mygliol 812N. (formulation 1-1)
   2) Soya oil. (formulation 1-2)
   3) Dimethicone 100 cps (formulation 1-3)
   4) Dimethicone 500 cps (formulation 1-4)
Formulation 2: Ibuprofen coated particles with the following formula were prepared by coacervation according to U.S. Patent No. 5,814,332. 83.4% by weight ibuprofen and 16.6% by weight of a mix of cellulose acetate phathalate, acid gelatin and additives including glutaraldehyde and sodium lauryl sulfate. These coated particles were dispersed in the following 4 lipophilic vehicles at a concentration of 25 wt. %:
   1) Mygliol^{®} 812N. (formulation 2-1)
   2) Soya oil. (formulation 2-2)
   3) Dimethicone 100 cps (formulation 2-3)
   4) Dimethicone 500 cps (formulation 2-4)
Formulation 3: Ibuprofen coated particles with the following formula were prepared by coating on a fluidized air bed: 80.0% by weight ibuprofen; 13.3% by weight of ethyl cellulose; and 6.7% by weight of hydroxypropylmethylcellulose. For this formulation, the ibuprofen had a particle size of 70 µm. For the fluidized bed preparation of the coated particles, all of the coating components were dissolved in a hydroalcoholic solution and then sprayed on the ibuprofen in a Wurster coating machine at a spray rate of 75-200 gm/minute at an inlet temperature of 4-16°C and used 200-600 cfm of process air. These coated particles were dispersed in the following four (4) lipophilic vehicles at a concentration of 25 wt. %:
   1) Mygliol^{®} 812N (formulation 3-1)
   2) Soya oil (formulation 3-2)
   3) Dimethicone 100 cps (formulation 3-3)
   4) Dimethicone 500 cps (formulation 3-4)

For each of these formulations, the taste masking and the appearance of the coated crystals were assessed over time (1h, 3h, 24h, 10 days, 14 days and 2 months after preparation). The formulations were tested for taste masking by two people. The taste masking is characterized by the following scale:
1 : good taste, no burning effect in the throat from the ibuprofen;
2 : slight burning effect noticeable, still acceptable;
3 : very pronounced and unacceptable burning effect;
4 : intolerable burning effect.

The appearance of the coated crystals was assessed by microscopic observation under a lens (x 10). The appearance of the coated crystals and of the coating was assessed by using the following scale:
A : particles intact;
B : slight swelling of the coating and appearance of a few loose crystals;
C : majority of crystals uncoated;
D : only uncoated crystals present.

The results obtained for each of the formulations are given in Table 1 below:

**TABLE 1**

| **Time** | **Formulation 1-1** | | **Formulation 1-2.** | | **Formulation 1-3** | | **Formulation 1-4** | |
|---|---|---|---|---|---|---|---|---|
| | **Taste** | Appearance | **Taste** | Appearance | **Taste** | Appearance | **Taste** | Appearance |
| 1 hour | 4 | D | 2 | NP | 1 | NP | NP | NP |
| 3 hours | NP | NP | 3 | NP | 1 | NP | NP | NP |
| 24 hours | NP | NP | 3 | NP | 1 | A | NP | NP |
| 10 days | NP | NP | 3 | NP | NP | NP | NP | NP |
| 14 days | NP | NP | NP | NP | 1 | NP | NP | NP |
| 2 months | 4 | D | 3-4 | C | 1 | A | NP | NP |
| | | | | | | | | |

| **Time** | **Formulation 2-1** | | **Formulation 2-2** | | **Formulation 2-3** | | **Formulation 2-4** | |
|---|---|---|---|---|---|---|---|---|
| | **Taste** | Appearance | **Taste** | Appearance | **Taste** | Appearance | **Taste** | Appearance |
| 1 hour | 2 | B | 2 | NP | 1 | NP | 1 | A |
| 3 hours | NP | NP | 2 | NP | 1 | NP | NP | NP |
| 24 hours | 4 | c | 3 | C | 1 | NP | 1 | A |
| 10 days | 4 | D | NP | NP | NP | NP | NP | NP |
| 14 days | NP | NP | NP | NP | 1 | NP | 1 | A |
| 2 months | NP | NP | 3 | C | 1 | A | 1 | A |
| | | | | | | | | |

| **Time** | **Formulation 3-1** | | **Formulation 3-2** | | **Formulation formulation 3-3 3-4** | | | |
|---|---|---|---|---|---|---|---|---|
| | **Taste** | Apperance | **Taste** | Appearance | **Taste** | Appearance | **Taste** | Appearance |
| 1 hour | 4 | D | 2 | NP | 1 | NP | 1 | NP |
| 3 hours | NP | NP | 3 | NP | 1 | NP | 1 | NP |
| 24 hours | 4 | D | 3 | C | 1 | NP | 1 | NP |
| 10 days | NP | NP | 3 | NP | NP | NP | NP | NP |
| 14 days | NP | NP | NP | NP | 1 | NP | 1 | NP |
| 2 months | 4 | D | 3 | C | 1 | A | NP | NP |

| | | | | | | | | |
|---|---|---|---|---|---|---|---|---|
| NP = Not Performed | | | | | | | | |

These examples show that dimethicone in which the ibuprofen is insoluble is the correct lipophilic vehicle. At 3 months time, the dimethicone formulations still exhibited excellent stability.

### Example 2: Coated Particles

Coated particles of ibuprofen were prepared by spraying a hydroalkanolic solution of ethyl cellulose and hydroxypropylmethylcellulose on 70 micron particles of ibuprofen in a air fluidized bed. The obtained coated particles present in the following composition were obtained using the conditions previously described.

| **Formula (66711)** | |
|---|---|
| **Component** | **Composition (% by wt.)** |
| Ibuprofen | 87.0 |
| Ethyl Cellulose | 8.7 |
| Hydroxypropylmethylcellulose | 4.3 |

### Characterization:

The size of the coated particles was measured by laser granulometry on a Mastersizer 2000 (Malvern) apparatus using mineral oil as dispersing medium. 10% by volume had a particle size of 76 µm or less (D10=76 µm), while 50% volume had a diameter of 152 µm or less (D50=152 µm) and 90% by volume of the particles had a size of 281 µm or less (D90=280 µm).

The stability of the different coated particles was measured in the different lipophilic vehicles used in Example 1. In order to give further evidence of the lipophilic vehicle selection, coated particles from above were dispersed in Mygliol^{®} 812N and silicon oil for 18 hours. The particle size was determined on each sample using a Mastersizer 2000. Results from this study are set forth in Table 2.

**TABLE 2**

| **Lipophilic vehicle** | **Time point** | |
|---|---|---|
| | **Initial** | **After 18 hours** |
| Mygliol 812N | D10 = 75 µm | D10 = 63 µm |
| | D50 = 154 µm | D50 = 128 µm |
| | D90 = 292 µm | D90 = 223 µm |
| Silicon oil | D10 = 77 µm | D10 = 79 µm |
| | D50 = 153 µm | D50 = 151 µm |
| | D90 = 285 µm | D90 = 289 µm |

The data demonstrate that coated particle integrity is best preserved using silicon oil.

### Coated particles stability:

The coated particles produced above were bulk packed in glass containers and were placed in stability enclosures, at controlled temperature and humidity: 40°C/75% RH.

The properties of coated particles were observed at production and after storing 1 and 3 months.

**TABLE 3**

| **Staility study (40°C / 75 %: RH)** | | | | |
|---|---|---|---|---|
| **Test** | **Specifications** | **To** | **1 month** | **3 months** |
| Appearance | White to yellowish powder | Comply | Comply | Comply |
| Taste (2 persons) | No or acceptable perception of bitterness and burning effect | Comply | Comply | Comply |
| Coating integrity | Uniform coating upon microscopic observation | Comply | Comply | Comply |
| Ibuprofen (mag/g) | 783 - 957 | 860 | 796 | 861 |
| Impurity IBAP (%) | < 0.3 | ND | ND | ND |

| | | | | |
|---|---|---|---|---|
| ND = Not detected. IBAP is a degradation product of ibuprofen. | | | | |

**TABLE 4**

| | | | |
|---|---|---|---|
| Dissolution profile of coated particles, fill formulations and capsules was monitored by applying the following dissolution test. An apparatus type III according to the USP was used (VANKEL BIODISS). Dissolution vessels were filled with 900 ml of pH 6.0 R2 buffer according the European Pharmacopea. Dissolution media heat is controlled at 37°C +/- 0.5°C during the whole test. A dip rate of 30 dips / minute was applied in order to mimic jaw strain onto the capsule. Samples of 10 ml each were collected at predetermined time points and the concentration of ibuprofen dissolved was measured using an HPLC / UV method. | | | |

| **Time (min)** | **Stability Study (40°C (75 % RH)** | | |
|---|---|---|---|
| | **To** | **1 month** | **3 months** |
| | **Dissolution kinetics (%)** | | |
| 0 | 0 | 0 | 0 |
| 5 | 13.7 | 14.5 | 12.1 |
| 10 | 23.8 | 22.6 | 22.3 |
| 20 | 32.8 | 30.8 | 40.5 |
| 30 | 37.7 | 36.3 | 53.1 |
| 60 | 47.8 | 46.0 | 71.2 |
| 90 | 52.8 | 53.7 | 77.6 |

| | | | |
|---|---|---|---|
| NP = Not Performed | | | |

The data indicates that the product is stable during at least 3 months. It is believed that the dissolution kinetics are accelerated with aging because of water absorption by the coating.

### Dispersion:

On the basis of the results obtained in the previous Examples, a dispersion was prepared which was intended to be filled in unitary packages such as "stick packs".

**TABLE 5**

| **Dispersion formula** | |
|---|---|
| **Component** | **Composition (% by wt.)** |
| Coated Ibuprofen (66711) | 20.9 |
| Dimethicone 100 cps | 69.5 |
| Colloidal Silica | 2.4 |
| Racemic Menthol | 0.1 |
| Xylitol 50 µm | 4.5 |
| Aspartame | 0.8 |
| Flavor(s) | 1.8 |

The dispersion was bulk packed in glass containers, which were placed in stability enclosures, at controlled temperature and humidity: 40°C/75% RH. The properties of coated particles were evaluated after storing for 1 month.

**TABLE 6**

| **Stability study (40°C / 75 % RH)** | | | |
|---|---|---|---|
| **Test** | **Specifications** | **To** | **1 month** |
| Appearance | White paste with solid particles dispersed in it | Comply | Comply |
| Taste (2 persons) | No or acceptable perception of bitterness and burning effect | Comply | Comply |
| Coating integrity | Uniform coating upon microscopic observation | Comply | Comply |
| Ibuprofen (mg/g) | 163.6 - 200.0 | 196.9 | 198.1 |
| Impurity IBAP (%) | < 0.3 | ND | ND |

| | | | |
|---|---|---|---|
| ND = Not detected. | | | |

### Chewable Soft Gel

The dispersion set forth in Table 2 was filled in a soft capsule using the rotary die process. The soft capsule is a chewable capsule. Gelatin was first melted in water in presence of glycerol and then a pre-dispersion of starch was added. After formation of the mixture, the gelatin mass was deaerated under vacuum.

**TABLE 7(a)**

| **Envelope Formula** | |
|---|---|
| **Component** | **Composition (% by wt.)** |
| Gelatin | 26.0 |
| Glycerol | 36.0 |
| Purified water | 22.0 |
| Modified starch (acetate) | 6.0 |
| Unbleached potato starch | 10.0 |

The envelope formula set forth in Table 7(a) was completed by adding sweetening composition set forth in Table 7(b).

**TABLE 7 (b)**

| **Sweetening Formula** | |
|---|---|
| **Component** | **Amount (g/kg of envelope)** |
| Aspartame | 2.5 |
| Racemic Menthol | 5.0 |
| Flavor(s) | 1.0 |

Capsules were prepared by an industrial apparatus based on the well known rotary die technique. Standard parameters of encapsulation were used. The weight of the filling dispersion was 1103 mg per capsule. Capsules were then dried.

### Capsules stability:

The capsules were bulk packed in glass containers, which were placed in stability enclosures, at controlled temperature and humidity: 40°C/75% RH. The properties of capsules were evaluated after production and after storing for 1 and 3 months.

**TABLE 8**

| **Stability study (40°C/75 % RH) - batch N°E08582** | | | | |
|---|---|---|---|---|
| **Test** | **Specifications** | **To** | **1 month** | **3 months** |
| Appearance | Opaque capsule filled with a white paste | Comply | Comply | Comply |
| Taste (2 persons) | No or acceptable perception of bitterness and burning effect | Comply | Comply | Comply |
| Coating integrity | Uniform coating upon microscopic observation | Comply | Comply | Comply |
| Ibuprofen (mg / caps) | 195.0 - 205.0 | 204.7 | 198.8 | 196.9 |

| Time (min) | | Dissolution kinetics (%) | | |
|---|---|---|---|---|
| 0 | | 0 | 0 | 0 |
| 5 | | 8.3 | 1.2 | 0.8 |
| 10 | | 10.6 | 2.7 | 2.1 |
| 20 | | 18.8 | 10.6 | 6.5 |
| 30 | | 22.2 | 17.3 | 12.5 |
| 60 | | 37.4 | 34.8 | 28.3 |
| 90 | | 46.8 | 46.0 | 41.2 |

### Example 3: Coated Particles

Coated particles of ibuprofen were prepared by hot spraying Precirol^{®} Ato 5 on particles of ibuprofen in a fluidized air bed equipment, according to a process described in U.S. Patent No. 6,194,005. The obtained coated particles present the following formula:

| **Formula (66712)** | |
|---|---|
| **Component** | **- Composition (%)** |
| Ibuprofen | 80.0 |
| Precirol Ato 5 | 20.0 |

### Characterization:

The size of the coated particles was measured by laser granulometry on a Mastersizer 2000 (Malvern) apparatus using a mineral oil as dispersing medium.
D10=62 µm
D50 = 139 µm
D90 = 285 µm

The stability of the different coated particles was measured in the different lipophilic vehicles used in Example 1. The stability study in dimethicone was confirmed at a concentration of 10% and has demonstrated a stability of the taste-masking for at least 3 months.

### Coated particles stability:

The coated particles were bulk packed in glass containers, which were placed in stability enclosures, at controlled temperature and humidity of 40°C and 75% relative humidity (RH). The properties of coated particles were evaluated after production and after 1 and 3 months of storage.

**TABLE 9**

| **Stability study (40°C/75% RH)** | | | | |
|---|---|---|---|---|
| | | **To** | **1 month** | **3 months** |
| Appearance | White to yellowish powder | Conform | Conform | Conform |
| Taste (2 persons) | No or acceptable perception of bitterness and burning effect | Conform | Conform | Conform |
| Coating integrity | Uniform coating upon microscopic observation | Conform | Conform | Conform |
| Ibuprofen (mg / g) | 720 - 880 | 779 | 745 | 779 |
| Impurity IBAP (%) | < 0.3 | ND | ND | ND |

| **Time (min)** | | **Dissolution kinetics (%)** | | |
|---|---|---|---|---|
| 0 | | 0 | 0 | 0 |
| 5 | | 2.9 | 3.8 | 4.1 |
| 10 | | 5.1 | 7.1 | 13.1 |
| 20 | | 9.0 | NP | 25.2 |
| 30 | | 12.1 | 15.1 | 32.6 |
| 60 | | 19.0 | 22.4 | 43.9 |
| 90 | | 25.3 | 28.4 | 50.7 |

| | | | | |
|---|---|---|---|---|
| NP = Not performed ND = Not detected | | | | |

The data indicate that the coated particles were stable for at least 3 months. After 3 months, the dissolution kinetics were accelerated due to an agglomeration of the coated particles, which had required a mechanical de-agglomeration, which may have damaged the coating.

### Dispersion:

On the basis of the results obtained in the previous examples, a dispersion was developed which is intended to be filled in unitary packages such as "stick packs".

**TABLE 10**

| **Dispersion Formula** | |
|---|---|
| **Component** | **Composition (%)** |
| Coated ibuprofen (66712) | 26.6 |
| Dimethicone 100 cps | 69.7 |
| Colloidal Silica | 2.7 |
| Sodium saccharine | 0.9 |
| Flavor(s) | 0.1 |

### Dispersion stability:

The dispersion was bulk packed in glass containers, which were placed in stability enclosures, at controlled temperature and humidity: 40°C / 75% RH. The properties of coated particles were evaluated after storing for 1 month.

**TABLE 11**

| **Stability study (40°C / 75 % RH)** | | | |
|---|---|---|---|
| **Test** | **Specifications** | **To** | **1 month** |
| Appearance | White paste with solid particles dispersed in it | Comply | Comply |
| Taste (2 persons) | No or acceptable perception of bitterness and burning effect | Comply | Comply |
| Coating integrity | Uniform coating upon microscopic observation | Comply | Comply |
| Ibuprofen (mg / g) | 191.5 - 234.1 | 215.6 | 212.1 |
| Impurity IBAP (%) | < 0.3 | ND | ND |

| | | | |
|---|---|---|---|
| ND = Not detected | | | |

### Chewable Capsules:

The dispersion obtained above was filled in a soft capsule using the rotary die process. The soft capsule was designed to be a chewable capsule. The gelatin was melted in water in the presence of glycerol and then a pre-dispersion of starch was added. After formation of the mixture, the gelatin mass was deaerated under vacuum.

**TABLE 12(a)**

| **Envelope Formula** | |
|---|---|
| **Component** | **Composition (% by wt.)** |
| Gelatin | 26.0 |
| Glycerol | 36.0 |
| Purified water | 22.0 |
| Modified starch (acetate) | 6.0 |
| Unbleached potato starch | 10.0 |

The envelope was completed with the following sweetening composition:

**TABLE 12(b)**

| **Sweetening formula** | |
|---|---|
| **Component** | **Composition (g / kg envelope)** |
| Aspartame | 2.5 |
| Racemic menthol | 5.0 |
| Flavor(s) | 1.0 |

Capsules were prepared by an industrial apparatus using the rotary die technique. Standard parameters of encapsulation were used. Each capsule contained 940 mg of dispersion per capsule. Capsules were then dried.

### Capsule stability:

The capsules were bulk packed in glass containers, placed in stability enclosures, at controlled temperature and humidity: 40°C/75% RH. The properties of the capsules were evaluated at time zero and after storing for 1 and 3 months.

**TABLE 13**

| **Stability study (40°C / 75 % RH) - batch N°E08563** | | | | |
|---|---|---|---|---|
| **Test** | **Specifications** | **To** | **1 month** | **3 months** |
| Appearance | Opaque capsule filled with a white paste | Comply | Comply | Comply |
| Taste (2 persons) | No or acceptable perception of bitterness and burning effect | Comply | Comply | Comply |
| Coating integrity | Uniform coating upon microscopic observation | Comply | Comply | Comply |
| Ibuprofen (mg / caps) | 180.0 - 190.0 | 188.7 | 179.4 | NP |

| **Time (min)** | | **Dissolution kinetics (%)** | | |
|---|---|---|---|---|
| 0 | | 0 | 0 | 0 |
| 5 | | 1.0 | 1.3 | 0.9 |
| 10 | | 1.5 | 2.5 | 1.8 |
| 20 | | 2.6 | 4.3 | 3.0 |
| 30 | | 3.6 | 5.7 | 4.1 |
| 60 | | 6.5 | 9.1 | 7.6 |
| 90 | | 9.7 | 12.3 | 11.2 |

### Example 4:

In this example, lipophilic vehicles with different solubilizing powers for dextromethorphan HBr were tested. The solubility of dextromethorphan HBr at 25°C was measured using an HPLC method in the following lipophilic vehicles:
1) Medium chain triglycerides sold by SASOL under the brand name Mygliol^{®} 812N provided a solubility of 120 µg/ml.
2) Soya oil provided a solubility of approx. 6 µg/ml;
3) Paraffin oil provided a solubility of approx. 1 µg/ml;
4) Dimethicone 100 silicone oil from RHODIA provided a solubility of less than 1 µg/ml.

Using a taste panel of 2 persons, it was determined that in aqueous buffer and lipophilic dispersions, dextromethorphan HBr taste is detected at concentrations around 0.1 mg/ml. Therefore, dimethicone, paraffin oil and soy oil should be the vehicles acceptable for such formulations. This was cross-checked by the following study.

The following formulations were prepared:

### Formulation 1:

Dextromethorphan HBr HBr coated particles with the following formula were prepared by coating using a fluidized air bed equipment as set forth above.
1) 76.9% by weight of dextromethorphan HBr;
2) 15.4% by weight of ethyl cellulose;
3) 7.7% by weight of hydroxypropylmethylcellulose.

These coated particles were dispersed at 25% weight/weight in the following 4 lipophilic vehicles:
1) Mygliol 812N (formulation 1-1)
2) Soya oil (formulation 1-2)
3) Paraffin oil (formulation 1-3)
4) Dimethicone 100 (formulation 1-4)

For each of these formulations, the taste masking and the appearance of the coated crystals were assessed over time (1 day and 3 days after preparation). The formulations were tested for taste masking by two people. The taste masking was characterized using the following scale:
1 : good taste;
2 : taste noticeable, still acceptable;
3 : very pronounced and unacceptable taste;
4 : intolerable taste.

The appearance of the coated crystals was assessed by microscopic observation under a lens (x 10). The appearance of the coated crystals and of the coating was assessed by using the following scale:
1 : crystals intact;
2 : slight swelling of the coating and appearance of a few loose crystals;
3 : majority of crystals uncoated;
4 : only uncoated crystals present

The results obtained for each of the formulations are given in Table 14.

**TABLE 14**

| **Time** | **Formulation 1-1** | | **Formulation 1-2** | | **Formulation 1-3** | | **Formulation 1-4** | |
|---|---|---|---|---|---|---|---|---|
| | **taste** | appearance | **taste** | appearance | **taste** | appearance | **taste** | appearance |
| 1 day | 2 | NP | 1 | NP | 1 | NP | 1 | NP |
| 3 days | 3 | NP | 1-2 | NP | 1 | NP | 1 | A |
| 1 month | 3 | B | 1-2 | A | 1 | A | 1 | A |

This experiment indicates that dimethicone (in which the dextromethorphan HBr is insoluble) is the best lipophilic vehicle. As back-up vehicles, paraffin oil and soya oil also provide acceptable results.

### Example 5: Coated Particles

Coated particles of dextromethorphan HBr were prepared by spraying an organic solution (90% by wt. acetone, 10% by wt. water) of ethyl cellulose and hydroxypropylmethylcellulose on particles of dextromethorphan HBr in a fluidized air bed equipment. The obtained coated particles consisted of the formula set forth in Table 15.

**TABLE 15**

| **Coated particles** | |
|---|---|
| **Component** | **Composition (% by wt.)** |
| Dextromethorphan HBr | 76.9 |
| Ethyl Cellulose | 15.4 |
| Hydroxypropylmethylcellulose | 7.7 |

One skilled in this art will appreciate that obtaining levels of active above 70% by wt. is unusual, especially if the coating is an effective taste masker.

### Characterization :

The size of the coated particles was measured by laser granulometry on a Mastersizer 2000 (Malvern) apparatus using mineral oil as the dispersing medium.
D10 = 96 µm
D50 = 166 µm
D90 = 280 µm

The stability of the coated particles was measured in the different lipophilic vehicles used in Example 4.

### Coated particles stability:

The coated particles were bulk packed in glass containers, and placed in stability enclosures, at controlled temperature and humidity: 40°C/75% RH. The data indicated that the coated particles were stable after at least 1 month of storage.

### Dispersion:

On the basis of the results obtained in the previous Examples, a dispersion was developed which is intended to be filled in unitary packages such as "stick packs."

**TABLE 16**

| **Dispersion** | |
|---|---|
| **Component** | **Amount (% by wt.)** |
| Coated dextromethorphan HBr | 4.22 |
| Silicon oil | 87.36 |
| Colloidal silica | 7.88 |
| Aspartame | 0.49 |
| Sucralose | 0.05 |

### Dispersion stability:

The dispersion was bulk packed in glass containers, which were placed in stability enclosures, at controlled temperature and humidity: 40°C/75% RH. Analysis of the data concluded that the dispersion was stable for at least 1 month under these storage conditions.

### Chewable Softgel:

The dispersion obtained above was filled in a chewable soft capsule using the rotary die process. The gelatin was first melted in water in presence of glycerol and then a pre-dispersion of starch was added. After formation of the mixture, the gelatin mass is deaerated under vacuum..

**TABLE 17(a)**

| **Envelope Formula** | |
|---|---|
| **Component** | **Composition (% by wt.)** |
| Gelatin | 26.0 |
| Glycerol | 36.0 |
| Purified water | 22.0 |
| Modified starch (acetate) | 6.0 |
| Unbleached potato starch | 10.0 |

The formula for the envelope was completed by adding the following sweetening composition.

**TABLE 17(b)**

| **Sweetening-Formula** | |
|---|---|
| **Component** | **Composition (g/kg envelope)** |
| Aspartame | 2.5 |
| Racemic Menthol | 5.0 |
| Flavor(s) | 1.0 |

Capsules were prepared by an industrial apparatus using the rotary die technique. Standard parameters of encapsulation were used. The weight of the filling dispersion was 924 mg per capsule. Capsules were then dried.

### Capsules stability:

The capsules were bulk packed in glass containers, which were placed in stability enclosures, at controlled temperature and humidity: 40°C/75% RH. The evaluation of these capsules indicated that they were stable for at least 1 month.

### INDUSTRIAL APPLICABILITY

The pharmaceutical companies are constantly in search of improved dosage forms. The present invention is particularly suitable for people having difficulties in swallowing such as infants and elderly people. The present invention has combined a number of features to provide a dosage form that is easily delivered and overcomes the problem of bad taste associated with many active ingredients.

## Claims

1. A dispersion of crystals or granules of active substance in a lipophilic vehicle, wherein said lipophilic vehicle has a solubulizing power for the active substance of less than 1.5 times the active substance concentration for which a taste is detected in water, and wherein said crystals or granules are coated by a coating for taste masking which is a hydrophilic coating and/or a lipophilic coating, and is selected from ethylcellulose, hydroxyethylcellulose, hydroxypropylmethylcellulose, hydroxypropylmethyl cellulose phthalate, methacrylic copolymer, polyethylene glycol behenate, glycerol palmitostearate, glycerol stearate, cetyl palmitate, glyceric macrogols, beeswaxes and gelucires, and mixtures thereof.

2. The dispersion according to claim 1, wherein the lipophilic vehicle is selected from vegetable oils, animal oils, tri-, di- or mono-glycerides of fatty acids, lipolysing oils, mineral oils, light waxes, Vaseline, silicone oil, dimethicones, mixtures of silicon oil and colloidal silica, and mixtures thereof.

3. The dispersion according to claim 1, wherein the lipophilic vehicle additionally includes one or more additives selected from sweeteners, flavorings, colorants, thickeners, dispersants, effervescent agents, super-disintegrants, lipophilic surfactants, hydrophilic surfactants, hydrosoluble agents, and mixtures thereof.

4. The dispersion according to claim 1, wherein the concentration of active substance in the dispersion is at most 75% by weight, based on the weight of the dispersion.

5. The dispersion according to claim 1, wherein the coating of the crystals or granules of active substance is selected from ethylcellulose, hydroxyethylcellulose, hydroxypropylmethylcellulose, hydroxypropylmethyl cellulose phthalate, methacrylic copolymer, polyethylene glycol behenate, glycerol palmitostearate, glycerol stearate, cetyl palmitate, glyceric macrogol beeswaxes, glycerol, PEG-32 stearate, PEG-32 palmitostearate, and mixtures thereof.

6. The dispersion according to claim 1, wherein the coating makes up between 5% and 50% by weight of the total weight of the coated granules or crystals.

7. The dispersion according to claim 1, wherein the average size of the coated crystals or granules is less than 300 µm.

8. The dispersion according to claim 1, wherein one or both of the granules of active substance and the coating for taste masking includes additives selected from colorants, sweeteners, flavorings, effervescent agents, super-disintegrants, lipophilic surfactants, hydrophilic surfactants, hydrosoluble agents, and mixtures thereof.

9. The dispersion according to claim 1, wherein said lipophilic vehicle is a dimethicone, said coating is a mixture of ethyl cellulose and hydroxypropylmethylcellulose, and said coating makes up 5% to 70% weight, of the total weight of the dispersion.

10. A chewable soft capsule including an outer envelope encapsulating the dispersion according to claim 5.

11. The soft capsule according to claim 10, wherein the outer envelope includes gelatin, a plasticizer, and at least one starch, optionally, amylum acetate.

12. The soft capsule according to any one of claims 10-11, wherein the outer envelope also includes an additive selected from sweeteners, flavorings, colorants, and mixtures thereof.

13. The soft capsule according to any one of claims 10-12, wherein the outer envelope includes 18-30% by weight of gelatin, 30-45% by weight of plasticizer, 3-12% by weight of starch or amylum acetate, up to 12% by weight of an unbleached starch, and water to 100%, all percentages by weight being based on the total weight of the outer envelope.

14. The soft capsule according to any one of claims 10-13, wherein the plasticizer is selected from polyols, glycerol, xylitol, sorbitol, polyglycerol, non-crystallizable solutions of sorbitol, glucose, fructose, glucose syrups, and mixtures thereof.

15. A process for the preparation of chewable soft capsules, including the steps of:
a) preparing an outer envelope;
b) as necessary, preparing granules of active substance;
c) coating crystals of active substance or granules prepared in step b), with a coating for taste masking which is a hydrophilic coating and/or a lipophilic coating, and is selected from ethylcellulose, hydroxyethylcellulose, hydroxypropylmethylcellulose, hydroxypropylmethyl cellulose phthalate, methacrylic copolymer, polyethylene glycol behenate, glycerol palmitostearate, glycerol stearate, cetyl palmitate, glyceric macrogols, beeswaxes and gelucires, and mixtures thereof;
d) dispersing additives in a lipophilic vehicle, wherein said lipophilic vehicle has a solubulizing power for the active substance of less than 1.5 times the active substance concentration for which a taste is detected in water, and optionally, milling the formed dispersion;
e) dispersing the coated crystals or granules in the lipophilic vehicle;
f) filling and sealing the capsules with said dispersion; and
g) drying the capsules.

## Patentansprüche

1. Eine Dispersion aus Kristallen oder Körnern einer aktiven Substanz in einer lipophilen Trägersubstanz, wobei besagte liphophile Trägersubstanz eine Solubilisierungskraft für die aktive Substanz von weniger als dem 1,5-Fachen der Konzentration der aktiven Substanz aufweist, bei welcher in Wasser ein Geschmack festgestellt wird, und wobei besagte Kristalle oder Körner mit einer Beschichtung für Geschmacksmaskierung beschichtet sind, welche eine hypdrophile Beschichtung und/oder eine lipophile Beschichtung ist und ausgewählt wird unter Ethylcellulose, Hydroxyethylcellulose, Hydroxypropylmethylcellulose, Hydroxypropylmethylcellulosephtalat, Methacryl-Copolymer, Polyethylenglycol-Behenat, Glycerinpalmitostearat, Glycerinstearat, Cetylpalmitat, Glycerin-Macrogolen, Bienenwachsen und Geluciren sowie Mischungen davon.

2. Die Dispersion gemäß Anspruch 1, wobei die lipophile Trägersubstanz ausgewählt wird unter Pflanzenölen, tierischen Ölen, Tri-, Di- oder Monoglyceriden von Fettsäuren, lipolysierenden Ölen, Mineralölen, leichten Wachsen, Vaseline, Silikonöl, Dimethiconen, Mischungen aus Silikonöl und Kolloid-Kieselerde sowie Mischungen davon.

3. Die Dispersion gemäß Anspruch 1, wobei die lipophile Trägersubstanz ferner einen oder mehrere Zusatzstoffe umfasst, ausgewählt unter Süßstoffen, Geschmacksstoffen, Farbstoffen, Verdickungsmitteln, Dispergiermitteln, Brausemitteln, Superzerfallsmitteln, lipophilen Tensiden, hydrophilen Tensiden, wasserlöslichen Agenzien sowie Mischungen davon.

4. Die Dispersion gemäß Anspruch 1, wobei die Konzentration der aktiven Substanz in der Dispersion höchstens 75 Gew.-% bezogen auf das Gewicht der Dispersion beträgt.

5. Die Dispersion gemäß Anspruch 1, wobei die Beschichtung der Kristalle oder Körner einer aktiven Substanz ausgewählt wird unter Ethylcellulose, Hydroxyethylcellulose, Hydroxypropylmethylcellulose, Hydroxypropylmethylcellulosephtalat, Methacryl-Copolymer, Polyethylenglycol-Behenat, Glycerinpalmitostearat, Glycerinstearat, Cetylpalmitat, Glycerin-Macrogol, Bienenwachsen, Glycerin, PEG-32-Stearat, PEG-32-Palmitostearat sowie Mischungen davon.

6. Die Dispersion gemäß Anspruch 1, wobei die Beschichtung zwischen 5 und 50 Gew.-% des Gesamtgewichts der beschichteten Körner oder Kristalle ausmacht.

7. Die Dispersion gemäß Anspruch 1, wobei die durchschnittliche Größe der beschichteten Kristalle oder Körner weniger als 300 µm beträgt.

8. Die Dispersion gemäß Anspruch 1, wobei die Körner einer aktiven Substanz und/oder die Beschichtung für Geschmacksmaskierung Zusatzstoffe enthalten, welche ausgewählt werden unter Farbstoffen, Süßstoffen, Geschmacksstoffen, Brausemitteln, Superzerfallsmitteln, lipophilen Tensiden, hydrophilen Tensiden, wasserlöslichen Agenzien sowie Mischungen davon.

9. Die Dispersion gemäß Anspruch 1, wobei besagte lipophile Trägersubstanz ein Dimethicon und besagte Beschichtung eine Mischung aus Ethylcellulose und Hydroxypropylmethylcellulose ist, und wobei besagte Beschichtung 5 bis 70 Gew.-% des Gesamtgewichts der Dispersion ausmacht.

10. Eine kaubare Weichkapsel umfassend eine äußere Hülle, welche die Dispersion gemäß Anspruch 5 einkapselt.

11. Die Weichkapsel gemäß Anspruch 10, wobei die äußere Hülle Gelatine, einen Weichmacher und mindestens eine Stärke und, wahlweise, Amylacetat umfasst.

12. Die Weichkapsel gemäß irgendeinem der Ansprüche 10-11, wobei die äußere Hülle ferner einen Zusatzstoff ausgewählt unter Süßstoffen, Geschmacksstoffen, Farbstoffen sowie Mischungen davon umfasst.

13. Die Weichkapsel gemäß irgendeinem der Ansprüche 10-12, wobei die äußere Hülle Folgendes umfasst: 18-30 Gew.-% Gelatine, 30-45 Gew.-% Weichmacher, 3-12 Gew.-% Stärke oder Amylacetat, bis zu 12 Gew.-% einer ungebleichten Stärke und Wasser zu 100 %, wobei alle Gewichtsprozentsätze auf das Gesamtgewicht der äußeren Hülle bezogen sind.

14. Die Weichkapsel gemäß irgendeinem der Ansprüche 10-13, wobei der Weichmacher ausgewählt wird unter Polyolen, Glycerin, Xylitol, Sorbitol, Polyglycerin, nichtkristallisierbaren Lösungen von Sorbitol, Glucose, Fructose, Glucosesirupen sowie Mischungen davon.

15. Ein Verfahren zur Herstellung kaubarer Weichkapseln, bestehend aus folgenden Schritten:
a) Herstellen einer äußeren Hülle;
b) gegebenenfalls Herstellen von Körnern einer aktiven Substanz;
c) Beschichten von Kristallen einer aktiven Substanz oder von in Schritt b) hergestellten Körnern mit einer Beschichtung für Geschmacksmaskierung, welche eine hypdrophile Beschichtung und/oder eine lipophile Beschichtung ist und ausgewählt wird unter Ethylcellulose, Hydroxyethylcellulose, Hydroxypropylmethylcellulose, Hydroxypropylmethylcellulosephtalat, Methacryl-Copolymer, Polyethylenglycol-Behenat, Glycerinpalmitostearat, Glycerinstearat, Cetylpalmitat, Glycerin-Macrogolen, Bienenwachsen und Geluciren sowie Mischungen davon.
d) Dispergieren von Zusatzstoffen in einer lipophilen Trägersubstanz, wobei besagte lipophile Trägersubstanz eine Solubilisierungskraft für die aktive Substanz von weniger als dem 1,5-Fachen der Konzentration der aktiven Substanz aufweist, bei welcher in Wasser ein Geschmack festgestellt wird, und wahlweise Mahlen der gebildeten Dispersion;
e) Dispergieren der beschichteten Kristalle oder Körner in der lipophilen Trägersubstanz;
f) Füllen und Versiegeln der Kapseln mit besagter Dispersion; und
g) Trocknen der Kapseln.

## Revendications

1. Dispersion de cristaux ou de granules d'une substance active dans un véhicule lipophile **caractérisée en ce que** ledit véhicule lipophile possède une action solubilisante par rapport à la substance active 1,5 fois inférieure à la concentration de la substance active pour laquelle un gout est détecté dans de l'eau, et **caractérisée en ce que** lesdits cristaux ou granules sont enrobés d'un enrobage servant à masquer le gout, celui-ci correspondant à un enrobage hydrophile et/ou lipophile, sélectionné à partir de l'éthylcellulose, l'hydroxyéthylcellulose, l'hydroxypropylméthylcellulose, l'hydroxypropylméthyl cellulose phthalate, le copolymère méthacrylique, le béhénate de polyéthylène glycol, le palmitostéarate de glycérol, le stéarate de glycérol, le palmitate de cétyle, les cires d'abeilles le Macrogol^{®} glycériques et Gelucires^{®}, et leurs mélanges.

2. Dispersion selon la revendication 1, **caractérisée en ce que** le véhicule lipophile est sélectionné à partir d'huiles végétales, d'huiles animales, de tri-, di- ou monoglycérides d'acides gras, d'huiles lipolysantes, d'huiles minérales, de cires légères, de Vaseline, d'huile de silicone, de diméthicones, de mélanges d'huile de silicone et de silice colloïdale, et leurs mélanges.

3. Dispersion selon la revendication 1, **caractérisée en ce que** le véhicule lipophile en outre inclut un ou plusieurs additifs sélectionnés parmi les édulcorants, les arômes, les colorants, les agents épaississants, les agents effervescents, les super-désintégrants, les tensio-actifs lipophiles, les tensio-actifs hydrophiles, les agents hydrosolubles, et leurs mélanges.

4. Dispersion selon la revendication 1, **caractérisée en ce que** la concentration de substances actives de la dispersion est au plus égale à 75% en poids, par rapport au poids de la dispersion.

5. Dispersion selon la revendication 1, **caractérisée en ce que** l'enrobage des cristaux ou des granules de la substance active est sélectionné à partir de l'éthylcellulose, l'hydroxyéthylcellulose, l'hydroxypropylméthylcellulose, l'hydroxypropylméthyl cellulose phthalate, le copolymère méthacrylique, le béhénate de polyéthylène glycol, le palmitostéarate de glycérol, le stéarate de glycérol, le palmitate de cétyle, les cires d'abeilles Macrogol^{®} glycériques, le glycérol, le stéarate PEG-32 le palmitostéarate PEG-32, et leurs mélanges.

6. Dispersion selon la revendication 1, **caractérisée en ce que** l'enrobage constitue entre 5% et 50% en poids, par rapport au poids total des granules ou cristaux enrobés.

7. Dispersion selon la revendication 1, **caractérisée en ce que** la dimension moyenne des cristaux ou granules enrobés est inférieure à 300 µm.

8. Dispersion selon la revendication 1, **caractérisée en ce que**, soit séparément, soit l'ensemble, granules de substances actives et enrobage servant à masquer le gout, inclut des additifs sélectionnés parmi les colorants, édulcorants, les arômes, les agents effervescents, les super-désintégrants, les tensio-actifs lipophiles, les tensio-actifs hydrophiles, les agents hydrosolubles, et leurs mélanges.

9. Dispersion selon la revendication 1, **caractérisée en ce que** ledit véhicule lipophile est un diméthicone, ledit enrobage est un mélange d'éthyle cellulose et d'hydroxypropylméthylcellulose, et ledit enrobage constitue entre 5% et 70% en poids par rapport au poids total de la dispersion.

10. Gélule molle à mâcher comprenant une enveloppe externe qui encapsule la dispersion selon la revendication 5.

11. Gélule molle à mâcher selon la revendication 10, **caractérisée en ce que** l'enveloppe externe inclut une gélatine, un plastifiant, et au moins un amidon, éventuellement l'acétate d'amylose.

12. Gélule molle à mâcher selon l'une quelconque des revendications 10 à 11, **caractérisée en ce que** l'enveloppe externe inclut aussi un additif sélectionné parmi les édulcorants, les arômes, les colorants et leurs mélanges.

13. Gélule molle à mâcher selon l'une quelconque des revendications 10 à 12, **caractérisée en ce que** l'enveloppe externe inclut 18 à 30 % en poids de gélatine, 30 à 45% en poids de plastifiant, 3 à 12 % en poids d'amidon ou d'acétate d'amylose, jusqu'à 12 % en poids d'un amidon non blanchi, et de l'eau à 100 %, tous les pourcentages poids étant par rapport au poids total de l'enveloppe externe.

14. Gélule molle à mâcher selon l'une quelconque des revendications 10 à 13, **caractérisée en ce que** le plastifiant est sélectionné parmi les polyols, le glycérol, le xylitol, le sorbitol, le polyglycérol, les solutions de sorbitol non cristallisables, le glucose, le fructose, les sirops de glucose, et leurs mélanges.

15. Procédé pour la préparation de gélules molles à mâcher, comprenant les étapes consistant à :
a) préparer une enveloppe externe ;
b) selon les besoins préparer des granules de substance active ;
c) enrober les cristaux ou granules de substance active préparés à l'étape b), avec un enrobage servant à masquer le gout et qui correspond à un enrobage hydrophile et/ou un enrobage lipophile, sélectionné à partir de l'éthylcellulose, l'hydroxyéthylcellulose, l'hydroxypropylméthylcellulose, l'hydroxypropylméthyl cellulose phthalate, le copolymère méthacrylique, le béhénate de polyéthylène glycol, le palmitostéarate de glycérol, le stéarate de glycérol, le palmitate de cétyle , les cires d'abeilles Macrogol^{®} glycériques et Gelucires^{®}, et leurs mélanges ;
d) disperser des additifs dans un véhicule lipophile, **caractérisée en ce que** ledit véhicule lipophile possède une action solubilisante par rapport à la substance active 1,5 fois inférieure à la concentration de la substance active pour laquelle un gout est détecté dans de l'eau, et éventuellement, broyer la dispersion formée ;
e) disperser les cristaux ou granules enrobés dans le véhicule lipophile ;
f) remplir et obturer les gélules de ladite dispersion ; et
g) sécher les gélules.
